# EUROPEAN PATENT SPECIFICATION

(11) **EP 4 080 440 B1**
(45) Date of publication and mention of the grant of the patent: **18.11.2026**
(21) Application number: 20902054.4
(22) Date of filing: 21.12.2020
(51) Int. Cl.: G06Q 30/06, A61B 5/00, A61B 10/00, G06Q 30/0282, G06Q 30/0601, G06Q 50/02, G16H 20/60, G16H 50/20, G16H 50/70

(54) **DEVICE AND METHOD FOR PROVIDING FODDER INFORMATION OPTIMIZED FOR INDIVIDUALS**
GERÄT UND VERFAHREN ZUM BEREITSTELLEN VON FÜR PERSONEN OPTIMIERTEN FUTTERMITTELINFORMATIONEN
DISPOSITIF ET PROCÉDÉ DE FOURNITURE D'INFORMATIONS DE FOURRAGE OPTIMISÉES POUR DES INDIVIDUS

(30) Priority: 20.12.2019 KR 20190171652; 18.12.2020 KR 20200178224
(43) Date of publication of application: 26.10.2022
(73) Proprietor: Seoul National University R & DB Foundation, Seoul 08826 (KR)
(72) Inventor: YEON, Seong Chan, Seoul 08741 (KR)
(74) Representative: dompatent
(86) International application number: PCT/KR2020/018736
(87) International publication number: WO 2021/125908

(56) References cited:
- WO-A2-2007/075932
- KR-A- 20150 133 978
- KR-A- 20180 078 458
- KR-A- 20180 107 630
- US-A1- 2014 272 028
- US-A1- 2019 251 861
- US-A1- 2019 252 058
- US-A1- 2019 290 172
- US-A1- 2019 295 440

## Description

### [Technical Field]

The present disclosure relates to a device and method for providing food information optimized for each individual.

### [Background Art]

Recently, as the number of pet households has rapidly increased, the need for a food recommendation service for pets is being strongly raised. As the number of types of food has rapidly increased along with an increase in the number of pet households, pet owner are always sensitive to food feeding. However, since it is difficult for the pet owners to determine whether the corresponding food is suitable for pets, it is common to purchase food based on brand awareness only.

However, the pets vary in types and breeds, and the nutrients that the pet should consume vary depending on its age, size, physical condition, disease, etc. Therefore, it is not easy for the pet owners to choose food that contains the necessary nutrients customized for their pets. Relevant prior art includes US 2019/290172 A1, US 2019/295440 A1, US 2019/251861 A1, US 2019/252058 A1.

Accordingly, a research on technology that recommends food optimized for each pet is continuing.

### [Disclosure]

### [Technical Problem]

The present disclosure provides a device and method for providing food information optimized for each individual.

### [Technical Solution]

In one aspect of the present disclosure, there is provided a food information providing device optimized for each individual comprising a data acquisition unit configured to acquire characteristic information and microbiome analysis result information of a target individual, and a processor configured to determine a food customized for the target individual based on the acquired characteristic information and microbiome analysis result information.

The characteristic information may include a type, a breed, a weight, an age, a sex, an obesity status, a disease status, a blood test result, and a neutering surgery of an individual.

The processor may be configured to determine nutrients required for the target individual based on the acquired characteristic information and microbiome analysis result information and determine a food composition capable of providing the determined nutrients.

The food composition may include a type, a quality, and a quantity of components of the food.

The processor may be configured to determine a health state of the target individual based on the acquired characteristic information and microbiome analysis result information, and when the determined health state is abnormal, determine types and a ratio of harmful bacteria and beneficial bacteria in the target individual using the microbiome analysis result information, determine types and an amount of beneficial bacteria required for the target individual, and determine the food composition considering the types and the amount of beneficial bacteria required for the target individual.

The food information providing device may further comprise an output unit configured to output information on the determined food.

The food information may include a food type, product information, and a dietary method.

The processor may be configured to use a food recommendation model trained to determine an optimal food customized for an individual based on characteristic information and microbiome analysis result information.

In another aspect of the present disclosure, there is provided a method of providing food information optimized for each individual, the method comprising acquiring characteristic information and microbiome analysis result information of a target individual, and determining a food customized for the target individual based on the acquired characteristic information and microbiome analysis result information.

The characteristic information may include a type, a breed, a weight, an age, a sex, an obesity status, a disease status, a blood test result, and a neutering operation of an individual.

Determining the food customized for the target individual may comprise determining nutrients required for the target individual based on the acquired characteristic information and microbiome analysis result information, and determining a food composition capable of providing the determined nutrients.

The food composition may include a type, a quality, and a quantity of components of the food.

Determining the food composition may comprise determining a health state of the target individual based on the acquired characteristic information and microbiome analysis result information, and when the determined health state is abnormal, determining types and a ratio of harmful bacteria and beneficial bacteria in the target individual using the microbiome analysis result information, determining types and an amount of beneficial bacteria required for the target individual, and determining the food composition considering the types and the amount of beneficial bacteria required for the target individual.

The method may further comprise outputting information on the determined food.

The food information may include a food type, product information, and a dietary method.

Determining the food customized for the target individual may comprise determining the food customized for the target individual using a food recommendation model trained to determine an optimal food customized for an individual based on characteristic information and microbiome analysis result information.

### [Advantageous Effects]

The present disclosure can recommend food optimized for each individual based on characteristics and a microbiome analysis result of individuals. Hence, a user can easily select food of individual.

### [Description of Drawings]

FIG. 1 illustrates a food information providing device optimized for each individual according to an exemplary embodiment.
FIG. 2 illustrates a food information providing device optimized for each individual according to an exemplary embodiment.
FIG. 3 illustrates a method of providing food information optimized for each individual according to an exemplary embodiment.

### [Mode for Invention]

Reference will now be made in detail to embodiments of the disclosure, examples of which are illustrated in the accompanying drawings. Wherever possible, the same reference numbers will be used throughout the drawings to refer to the same or like parts. It will be noted that a detailed description of known arts will be omitted if it is determined that the detailed description of the known arts can obscure the embodiments of the disclosure.

For respective steps, respective steps may be performed in a different order from the stated order unless the context clearly indicates a specific order. That is, the respective steps may be performed in the same order as specified, or may be performed substantially simultaneously, or may be performed in the reverse order.

The terms to be described later are terms defined in consideration of functions in the present disclosure and may vary depending on intentions or customs, etc. of users or operators. Therefore, the definition should be made based on the contents throughout the present disclosure.

The terms including an ordinal number such as first, second, etc. may be used to describe various components, but the components are not limited by such terms. The terms are used only for the purpose of distinguishing one component from other components. A singular expression can include a plural expression as long as it does not have an apparently different meaning in context. In the present disclosure, terms "include" and "have" should be understood to be intended to designate that illustrated features, numbers, steps, operations, components, parts or combinations thereof are present and not to preclude the existence of one or more different features, numbers, steps, operations, components, parts or combinations thereof, or the possibility of the addition thereof.

In the present disclosure, the classification of components is merely classified based on the main function for which each component is responsible. That is, two or more components may be combined into one component, or one component may be divided into two or more components for each more subdivided function. Each of the components may additionally perform all or part of function of another component in addition to the main function for which each component is responsible, and part of the main function of each component may be exclusively performed by another component. Each component may be implemented as hardware or software, or may be implemented as a combination of hardware and software.

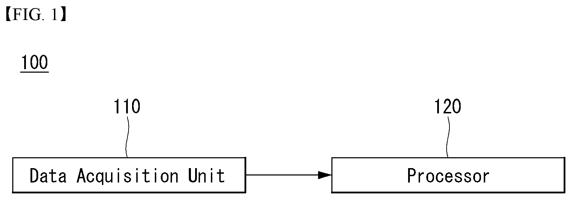

FIG. 1 illustrates a food information providing device optimized for each individual according to an exemplary embodiment. A food information providing device 100 optimized for each individual (hereinafter, referred to as a 'food information providing device') illustrated in FIG. 1 is a device for providing food information optimized for a target individual based on characteristics and a microbiome analysis result of the target individual, and may be mounted on an electronic device or may be surrounded by a housing and formed as a separate device. Examples of the electronic device may include a personal computer, a mobile phone, a smartphone, a tablet, a laptop, a personal digital assistant (PDA), a portable multimedia player (PMP), a navigation device, an MP3 player, a digital camera, a wearable device, and the like. Examples of the wearable device may include a glasses type, a wrist watch type, a wristband type, a ring type, an earring type, a belt type, a necklace type, an ankle band type, a thigh band type, a forearm band type, and the like. The electronic device is not limited to the examples described above, and the wearable device is not limited to the examples described above.

Referring to FIG. 1, the food information providing device 100 according to an exemplary embodiment may include a data acquisition unit 110 and a processor 120.

The data acquisition unit 110 may acquire characteristic information of the target individual and information of microbiome analysis result of the target individual. Herein, the individual may include human and animal that consume food (e.g., feed, fodder, etc.), and the characteristic information of the individual may include a type (e.g., human, dog, cat, etc.), breed, weight, age, sex, an obesity status, a disease status, a blood test result, a neutering surgery of individual, and the like.

For example, the data acquisition unit 110 may acquire the characteristic information of the target individual from a hospital (including a veterinary hospital) server that measures and/or stores characteristic information of individuals, and acquire the microbiome analysis result of the target individual from a company server that performs microbiome analysis of individuals. In this instance, the data acquisition unit 110 may use wired and wireless communication technology. Herein, the wireless communication technology may include Bluetooth communication, Bluetooth Low Energy (BLE) communication, near field communication (NFC), WLAN communication, Zigbee communication, Infrared Data Association (IrDA) communication, Wi-Fi direct (WFD) communication, ultra-wideband (UWB) communication, Ant+ communication, WiFi communication, radio frequency identification (RFID) communication, 3G communication, 4G communication, 5G communication, and the like, but is not limited thereto.

As another example, the data acquisition unit 110 may receive the characteristic information and the microbiome analysis result information of the target individual from the user through a predetermined input means to acquire the characteristic information of the target individual and the microbiome analysis result information of the target individual.

The processor 120 may control an overall operation of the food information providing device 100.

The processor 120 may determine an optimal food for the target individual based on the characteristic information and the microbiome analysis result information of the target individual, and output information on the determined food through an output means. The food information may include food type, product information, dietary methods, etc., and the product information may include product name, manufacturer, food components, nutrients, additives, etc.

According to an embodiment, the processor 120 may determine an optimal food composition customized for the target individual based on the characteristic information and the microbiome analysis result information of the target individual. The food composition may include type, quality, quantity, etc. of components of food.

Nutrients required for each individual may vary depending on the characteristics of each individual, such as the type, breed, weight, age, sex, obesity status, disease status, blood test result, neutering surgery, etc. of each individual and/or the microorganisms present in each individual. Accordingly, according to an embodiment, the processor 120 may determine nutrients required for the target individual based on the acquired characteristic information and the microbiome analysis result information of the target individual, and may determine an optimal food composition that can provide the determined nutrients, for example, type, quality, quantity, etc. of components of food. In this instance, the processor 120 may consider components of food that cannot be consumed for each type of individuals.

For example, the processor 120 may determine whether a health state of the target individual is normal or abnormal based on the characteristic information and the microbiome analysis result information of the target individual. If it is determined that the health state of the target individual is abnormal, the processor 120 may determine types and a ratio of harmful bacteria and beneficial bacteria in the target individual using the microbiome analysis result information, and determine types and an amount of beneficial bacteria required for the target individual. In this instance, the processor 120 may consider type and ratio of beneficial bacteria that exist in an individual with a normal health state. Further, the processor 120 may determine an optimal food composition customized for the target individual considering the types and amount of beneficial bacteria required for the target individual.

The processor 120 may determine a microbiome product including beneficial bacteria required for the target individual and output information of the determined microbiome product through an output means. The microbiome product information may include a product name, a manufacturer, and types and an amount of microorganisms, and the like. Multiple microbiome product information may be previously stored in a database inside or outside the food information providing device 100, and the processor 120 may search the database to determine a microbiome product customized for the target individual.

When the optimal food composition customized for the target individual is determined, the processor 120 may determine the optimal food customized for the target individual based on the determined food composition. In this instance, the processor 120 may consider quality and quantity of food ingredients that help food components. Multiple food information may be previously stored in a database inside or outside the food information providing device 100, and the processor 120 may search the database to determine optimal food for the target individual.

According to an embodiment, the processor 120 may determine the optimal food for the target individual using a food recommendation model.

The food recommendation model may be a machine learning model trained to determine an optimal food customized for individual based on characteristic information and microbiome analysis result information of individuals. For example, the food recommendation model may be previously generated through machine learning based on learning data including characteristics and microbiome analysis result of each individual for various individuals, and food corresponding to them. The machine learning model may include artificial neural network, decision tree, genetic algorithm, genetic programming, K-nearest neighbor, radial basis function network, random forest, support vector machine, and deep-learning, and the like.

The processor 120 may periodically update the food recommendation model. For example, the processor 120 may continue to collect additional learning data, for example, actual data on the target individual (characteristics and microbiome analysis result of the target individual and the corresponding optimal food), and periodically additionally learn it using the collected additional learning data to update the food recommendation model. A performance of the food recommendation model can be improved by continuously updating the food recommendation model. That is, as the number of usages of the food recommendation model increases, it can learn with more data. Therefore, the performance of the food recommendation model can be further improved by an increase in the number of usages.

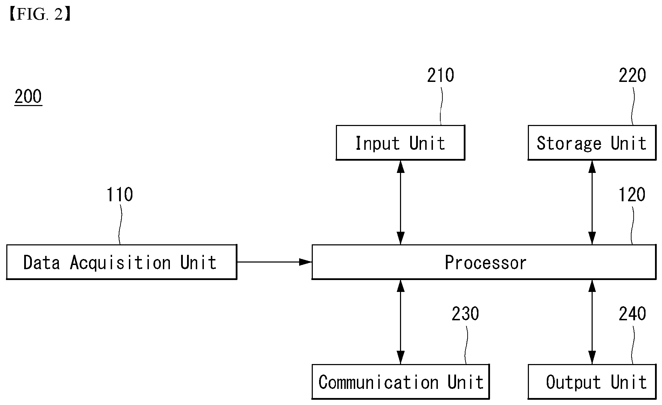

FIG. 2 illustrates a food information providing device optimized for each individual according to an exemplary embodiment.

Referring to FIG. 2, a food information providing device 200 may include a data acquisition unit 110, a processor 120, an input unit 210, a storage unit 220, a communication unit 230, and an output unit 240. Since the data acquisition unit 110 and the processor 120 are the same as those described above with reference to FIG. 1, a detailed description thereof is omitted.

The input unit 210 may receive various operating signals and information from a user. According to an embodiment, the input unit 210 may include a key pad, a dome switch, a touch pad, a jog wheel, a jog switch, an H/W button, and the like. In particular, when the touch pad forms an inter-layer structure with a display, it may be referred to as a touch screen.

The storage unit 220 may store programs or commands for an operation of the food information providing device 200, and store input data and processed data of the food information providing device 200, data required to determine food optimized for a target individual, and the like.

The storage unit 220 may include at least one type of storage medium such as, a flash memory type, a hard disk type, a multimedia card micro type, a card type memory (e.g., SD or XD memory, etc.), a random access memory (RAM), a static random access memory (SRAM), a read only memory (ROM), an electrically erasable programmable read only memory (EEPROM), a programmable read only memory (PROM), a magnetic memory, a magnetic disk, or an optical disk. The food information providing device 200 may operate an external storage medium such as web storage that performs a storage function of the storage unit 220 on the Internet.

The communication unit 230 may communicate with an external device. For example, the communication unit 230 may transmit input data, stored data, processed data, etc. of the food information providing device 200 to the external device, or receive, from the external device, various data required to determine food optimized for the target individual.

The communication unit 230 may communicate with the external device using wired and wireless communication technology. The wireless communication technology may include Bluetooth communication, Bluetooth Low Energy (BLE) communication, near field communication (NFC), WLAN communication, Zigbee communication, Infrared Data Association (IrDA) communication, Wi-Fi direct (WFD) communication, ultra-wideband (UWB) communication, Ant+ communication, WiFi communication, radio frequency identification (RFID) communication, 3G communication, 4G communication, 5G communication, and the like, but is merely an example and is not limited thereto.

The output unit 240 may output input data, stored data, processed data, etc. of the food information providing device 200. According to an embodiment, the output unit 240 may output acquired characteristic information and microbiome analysis result information of the target individual, and optimal food composition information, optimal food information, and microbiome product information determined based on them through at least one method of an auditory method, a visual method, and a tactile method. To the end, the output unit 240 may include a display, a speaker, a vibrator, and the like.

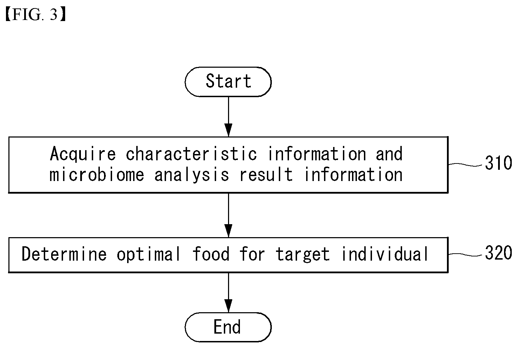

FIG. 3 illustrates a method of providing food information optimized for each individual according to an exemplary embodiment. The method of providing food information illustrated in FIG. 3 may be performed by the food information providing device 100 or 200 of FIG. 1 or 2.

Referring to FIG. 3, a food information providing device may acquire characteristic information of a target individual and microbiome analysis result information of the target individual, in 310.

For example, the food information providing device may acquire the characteristic information of the target individual from a hospital (including a veterinary hospital) server that measures and/or stores characteristic information of individuals, and acquire the microbiome analysis result of the target individual from a company server that performs microbiome analysis of individuals.

As another example, the food information providing device may receive the characteristic information and the microbiome analysis result information of the target individual from a user through a predetermined input means to acquire the characteristic information of the target individual and the microbiome analysis result information of the target individual.

The food information providing device may determine an optimal food for the target individual based on the characteristic information and the microbiome analysis result information of the target individual, and output information on the determined food, in 320. The food information may include food type, product information, dietary methods, etc., and the product information may include product name, manufacturer, food components, nutrients, additives, etc.

According to an embodiment, the food information providing device may determine an optimal food composition customized for the target individual based on the characteristic information and the microbiome analysis result information of the target individual. The food composition may include type, quality, quantity, etc. of components of food. For example, the food information providing device may determine nutrients required for the target individual based on the acquired characteristic information and the microbiome analysis result information of the target individual, and may determine an optimal food composition that can provide the determined nutrients, for example, type, quality, quantity, etc. of components of food. In this instance, the food information providing device may consider components of food that cannot be consumed for each type of individuals.

For example, the food information providing device may determine whether a health state of the target individual is normal or abnormal based on the characteristic information and the microbiome analysis result information of the target individual. If it is determined that the health state of the target individual is abnormal, the food information providing device may determine types and a ratio of harmful bacteria and beneficial bacteria in the individual using the microbiome analysis result information, and determine the types and amount of beneficial bacteria required for the target individual. Further, the food information providing device may determine an optimal food composition customized for the target individual considering the type and ratio of beneficial bacteria required for the target individual.

The food information providing device may determine a microbiome product including beneficial bacteria required for the target individual and output information of the determined microbiome product. The microbiome product information may include a product name, a manufacturer, and types and amount of microorganisms, and the like.

When the optimal food composition customized for the target individual is determined, the food information providing device may determine the optimal food customized for the target individual based on the determined food composition. In this instance, the food information providing device may consider quality and quantity of food ingredients that help food components.

According to another embodiment, the food information providing device may determine the optimal food for the target individual based on the characteristic information and the microbiome analysis result information of the target individual, and a food recommendation model.

The food recommendation model may be a machine learning model trained to determine an optimal food customized for individual based on characteristic information and microbiome analysis result information of individuals. For example, the food recommendation model may be previously generated through machine learning based on learning data including characteristics and microbiome analysis result of each individual for various individuals, and food corresponding to them.

The device and method for providing food information according to an exemplary embodiment may complete may complete an optimal food composition customized for the target individual based on the characteristic information and the microbiome analysis result information of the target individual, determine food close to the completed food composition, and provide information on the food. In this instance, microbiome product information required for the target individual may be provided together, if necessary or desired. The completed food composition may be used to newly product food of the target individual.

The device and method for providing food information according to an exemplary embodiment may be used in a field of animal insurance to set an insurance rate. That is, the food information providing device and method may cause disease decrease/increase, etc. based on food customized for each individual, and may be used to calculate the insurance rate based on this result. For example, animals that are fed based on the food information providing device and method according to an exemplary embodiment may be given a relatively lower insurance rate than other animals.

The embodiments described above may be implemented using a computer-readable medium with programs recorded thereon to perform various methods presented herein. The computer-readable medium may include all kinds of recording devices storing data that is readable by a computer system. Examples of the computer-readable medium may include ROM, RAM, CD-ROM, a magnetic tape, a floppy disk, an optical disc, and the like. The computer-readable medium may be distributed into computer systems connected via networks and may be executed by being written in computer-readable codes through a distribution method.

## Claims

1. A food information providing device optimized for each individual, comprising:
a data acquisition unit (110) configured to acquire characteristic information including a neutering surgery status and microbiome analysis result information of a target individual; and
a processor (120) configured to determine a food customized for the target individual based on the acquired characteristic information and microbiome analysis result information,
wherein the processor (120) is configured to determine an optimal food customized, using a food recommendation model trained, for the target individual based on the characteristic information and the microbiome analysis result information of the target individual, and
wherein the food recommendation model trained is previously generated through machine learning based on learning data including characteristics including a neutering surgery status, microbiome analysis result, and food corresponding to the characteristics and the microbiome analysis result, of each individual for various individuals.

2. The food information providing device of claim 1, wherein the characteristic information includes a type, a breed, a weight, an age, a sex, an obesity status, a disease status, and a blood test result.

3. The food information providing device of claim 1, wherein the processor (120) is configured to determine nutrients required for the target individual based on the acquired characteristic information and microbiome analysis result information and determine a food composition capable of providing the determined nutrients.

4. The food information providing device of claim 3, wherein the food composition includes a type, a quality, and a quantity of components of the food.

5. The food information providing device of claim 3, wherein the processor (120) is configured to:
determine a health state of the target individual based on the acquired characteristic information and microbiome analysis result information; and
when the determined health state is abnormal, determine types and a ratio of harmful bacteria and beneficial bacteria in the target individual using the microbiome analysis result information, determine types and an amount of beneficial bacteria required for the target individual, and determine the food composition considering the types and the amount of beneficial bacteria required for the target individual.

6. The food information providing device of claim 1, further comprising:
an output unit (240) configured to output information on the determined food.

7. The food information providing device of claim 6, wherein the food information includes a food type, product information, and a dietary method.

8. A method of providing food information optimized for each individual, the method comprising:
acquiring (310) characteristic information including a neutering surgery status and microbiome analysis result information of a target individual; and
determining (320) a food customized for the target individual based on the acquired characteristic information and microbiome analysis result information,
wherein the determining determines an optimal food customized, using a food recommendation model trained, for the target individual based on the characteristic information and the microbiome analysis result information of the target individual, and
wherein the food recommendation model trained is previously generated through machine learning based on learning data including characteristics including a neutering surgery status, microbiome analysis result, and food corresponding to the characteristics and the microbiome analysis result, of each individual for various individuals.

9. The method of claim 8, wherein the characteristic information includes a type, a breed, a weight, an age, a sex, an obesity status, a disease status, and a blood test result.

10. The method of claim 8, wherein the determining (320) the food customized for the target individual comprises:
determining nutrients required for the target individual based on the acquired characteristic information and microbiome analysis result information; and
determining a food composition capable of providing the determined nutrients.

11. The method of claim 10, wherein the food composition includes a type, a quality, and a quantity of components of the food.

12. The method of claim 10, wherein determining (320) the food composition comprises:
determining a health state of the target individual based on the acquired characteristic information and microbiome analysis result information; and
when the determined health state is abnormal, determining types and a ratio of harmful bacteria and beneficial bacteria in the target individual using the microbiome analysis result information, determining types and an amount of beneficial bacteria required for the target individual, and determining the food composition considering the types and the amount of beneficial bacteria required for the target individual.

13. The method of claim 8, further comprising:
outputting information on the determined food.

14. The method of claim 13, wherein the food information includes a food type, product information and a dietary method.

## Patentansprüche

1. Vorrichtung zur Bereitstellung von Nahrungsinformationen, die für jedes Individuum optimiert ist, umfassend:
eine Datenerfassungseinheit (110), die so konfiguriert ist, dass sie charakteristische Informationen einschließlich eines Kastrationsoperationsstatus und Informationen als Ergebnis einer Mikrobiomanalyse eines Zielindividuums erfassen kann; und
einen Prozessor (120), der so konfiguriert ist, dass er anhand der erfassten charakteristischen Informationen und Informationen als Ergebnis der Mikrobiomanalyse eine Nahrung bestimmen kann, die für das Zielindividuum maßgeschneidert ist,
wobei der Prozessor (120) so konfiguriert ist, dass er unter Verwendung eines trainierten Nahrungsempfehlungsmodells anhand der charakteristischen Informationen und Informationen als Ergebnis der Mikrobiomanalyse des Zielindividuums eine für das Zielindividuum maßgeschneiderte optimale Nahrung bestimmen kann, und
wobei das trainierte Nahrungsempfehlungsmodell vorher durch Maschinenlernen auf der Basis von Lerndaten erzeugt wurde, die für verschiedene Individuen jeweils Merkmale einschließlich eines Kastrationsoperationsstatus, ein Ergebnis einer Mikrobiomanalyse und eine Nahrung umfassen, die den Merkmalen und dem Ergebnis der Mikrobiomanalyse des jeweiligen Individuums entspricht.

2. Vorrichtung zur Bereitstellung von Nahrungsinformationen gemäß Anspruch 1, wobei die charakteristischen Informationen einen Typ, eine Rasse, ein Gewicht, ein Alter, ein Geschlecht, einen Adipositasstatus, einen Krankheitsstatus und ein Bluttestergebnis umfassen.

3. Vorrichtung zur Bereitstellung von Nahrungsinformationen gemäß Anspruch 1, wobei der Prozessor (120) so konfiguriert ist, dass er anhand der erfassten charakteristischen Informationen und Informationen als Ergebnis der Mikrobiomanalyse Nährstoffe bestimmen kann, die für das Zielindividuum erforderlich sind, und dass er eine Nahrungszusammensetzung bestimmen kann, die die bestimmten Nährstoffe liefern kann.

4. Vorrichtung zur Bereitstellung von Nahrungsinformationen gemäß Anspruch 3, wobei die Nahrungszusammensetzung einen Typ, eine Qualität und eine Menge von Komponenten der Nahrung umfasst.

5. Vorrichtung zur Bereitstellung von Nahrungsinformationen gemäß Anspruch 3, wobei der Prozessor (120) so konfiguriert ist, dass er:
anhand der erfassten charakteristischen Informationen und Informationen als Ergebnis der Mikrobiomanalyse einen Gesundheitszustand des Zielindividuums bestimmen kann; und
wenn der bestimmte Gesundheitszustand anomal ist, Typen und ein Verhältnis von schädlichen Bakterien und nützlichen Bakterien in dem Zielindividuum bestimmen kann, indem er die Informationen als Ergebnis der Mikrobiomanalyse verwendet, Typen und eine Menge von nützlichen Bakterien, die für das Zielindividuum erforderlich sind, bestimmen kann, und in Anbetracht der für das Zielindividuum erforderlichen Typen und Menge von nützlichen Bakterien die Nahrungszusammensetzung bestimmen kann.

6. Vorrichtung zur Bereitstellung von Nahrungsinformationen gemäß Anspruch 1, weiterhin umfassend:
eine Ausgabeeinheit (240), die so konfiguriert ist, dass sie Informationen zu der bestimmten Nahrung ausgeben kann.

7. Vorrichtung zur Bereitstellung von Nahrungsinformationen gemäß Anspruch 6, wobei die Nahrungsinformationen einen Nahrungstyp, Produktinformationen und eine Ernährungsmethode umfassen.

8. Verfahren zur Bereitstellung von Nahrungsinformationen, die für jedes Individuum optimiert sind, wobei das Verfahren umfasst:
das Erfassen (310) von charakteristischen Informationen einschließlich eines Kastrationsoperationsstatus und von Informationen als Ergebnis einer Mikrobiomanalyse eines Zielindividuums; und
das Bestimmen (320) einer Nahrung, die für das Zielindividuum maßgeschneidert ist, anhand der erfassten charakteristischen Informationen und Informationen als Ergebnis der Mikrobiomanalyse,
wobei bei dem Bestimmen unter Verwendung eines trainierten Nahrungsempfehlungsmodells anhand der charakteristischen Informationen und der Informationen als Ergebnis der Mikrobiomanalyse des Zielindividuums eine für das Zielindividuum maßgeschneiderte optimale Nahrung bestimmt wird und
wobei das trainierte Nahrungsempfehlungsmodell vorher durch Maschinenlernen auf der Basis von Lerndaten erzeugt wurde, die für verschiedene Individuen jeweils Merkmale einschließlich eines Kastrationsoperationsstatus, ein Ergebnis einer Mikrobiomanalyse und eine Nahrung umfassen, die den Merkmalen und dem Ergebnis der Mikrobiomanalyse des jeweiligen Individuums entspricht.

9. Verfahren gemäß Anspruch 8, wobei die charakteristischen Informationen einen Typ, eine Rasse, ein Gewicht, ein Alter, ein Geschlecht, einen Adipositasstatus, einen Krankheitsstatus und ein Bluttestergebnis umfassen.

10. Verfahren gemäß Anspruch 8, wobei das Bestimmen (320) der Nahrung, die für das Zielindividuum maßgeschneidert ist, Folgendes umfasst:
das Bestimmen von Nährstoffen, die für das Zielindividuum erforderlich sind, anhand der erfassten charakteristischen Informationen und Informationen als Ergebnis der Mikrobiomanalyse; und
das Bestimmen einer Nahrungszusammensetzung, die die bestimmten Nährstoffe liefern kann.

11. Verfahren gemäß Anspruch 10, wobei die Nahrungszusammensetzung einen Typ, eine Qualität und eine Menge von Komponenten der Nahrung umfasst.

12. Verfahren gemäß Anspruch 10, wobei das Bestimmen (320) der Nahrungszusammensetzung Folgendes umfasst:
das Bestimmen eines Gesundheitszustands des Zielindividuums anhand der erfassten charakteristischen Informationen und Informationen als Ergebnis der Mikrobiomanalyse; und
wenn der bestimmte Gesundheitszustand anomal ist, das Bestimmen von Typen und eines Verhältnisses von schädlichen Bakterien und nützlichen Bakterien in dem Zielindividuum unter Verwendung der Informationen als Ergebnis der Mikrobiomanalyse, das Bestimmen von Typen und einer Menge von nützlichen Bakterien, die für das Zielindividuum erforderlich sind, und das Bestimmen der Nahrungszusammensetzung in Anbetracht der für das Zielindividuum erforderlichen Typen und Menge von nützlichen Bakterien.

13. Verfahren gemäß Anspruch 8, weiterhin umfassend:
das Ausgeben von Informationen zu der bestimmten Nahrung.

14. Verfahren gemäß Anspruch 13, wobei die Nahrungsinformationen einen Nahrungstyp, Produktinformationen und eine Ernährungsmethode umfassen.

## Revendications

1. Dispositif de fourniture d'informations sur les aliments, optimisé pour chaque individu, comprenant :
une unité d'acquisition de données (110) configurée pour acquérir des informations caractéristiques incluant un statut de stérilisation chirurgicale et des informations sur les résultats de l'analyse du microbiome d'un individu cible ; et
un processeur (120) configuré pour déterminer un aliment personnalisé pour l'individu cible sur la base des informations caractéristiques acquises et des informations sur les résultats de l'analyse du microbiome acquises,
dans lequel le processeur (120) est configuré pour déterminer, en utilisant un modèle de recommandation alimentaire entraîné, un aliment optimal personnalisé pour l'individu cible sur la base des informations caractéristiques et des informations sur les résultats de l'analyse du microbiome de l'individu cible, et
dans lequel le modèle de recommandation alimentaire entraîné a été généré au préalable par apprentissage automatique sur la base de données d'apprentissage incluant, pour chacun de divers individus, des caractéristiques comprenant un statut de stérilisation chirurgicale ; un résultat de l'analyse du microbiome ; et des aliments correspondant aux caractéristiques et au résultat de l'analyse du microbiome de cet individu.

2. Dispositif de fourniture d'informations sur les aliments selon la revendication 1, dans lequel les informations caractéristiques comprennent un type, une race, un poids, un âge, un sexe, un statut d'obésité, un statut de maladie et un résultat d'analyse sanguine.

3. Dispositif de fourniture d'informations sur les aliments selon la revendication 1, dans lequel le processeur (120) est configuré pour déterminer des nutriments nécessaires à l'individu cible sur la base des informations caractéristiques acquises et des informations sur les résultats de l'analyse du microbiome acquises, et pour déterminer une composition alimentaire capable de fournir les nutriments déterminés.

4. Dispositif de fourniture d'informations sur les aliments selon la revendication 3, dans lequel la composition alimentaire comprend un type, une qualité et une quantité de composants de l'aliment.

5. Dispositif de fourniture d'informations sur les aliments selon la revendication 3, dans lequel le processeur (120) est configuré pour :
déterminer un état de santé de l'individu cible sur la base des informations caractéristiques acquises et des informations sur les résultats de l'analyse du microbiome acquises ; et
lorsque l'état de santé déterminé est anormal, déterminer des types de bactéries nocives et de bactéries bénéfiques et un rapport entre ces bactéries chez l'individu cible en utilisant les informations sur les résultats de l'analyse du microbiome, déterminer des types et une quantité de bactéries bénéfiques nécessaires à l'individu cible, et déterminer la composition alimentaire en tenant compte des types et de la quantité de bactéries bénéfiques nécessaires à l'individu cible.

6. Dispositif de fourniture d'informations sur les aliments selon la revendication 1, comprenant en outre :
une unité de sortie (240) configurée pour fournir des informations sur l'aliment déterminé.

7. Dispositif de fourniture d'informations sur les aliments selon la revendication 6, dans lequel les informations sur les aliments comprennent un type d'aliment, des informations sur le produit et un mode d'alimentation.

8. Procédé de fourniture d'informations sur les aliments optimisées pour chaque individu, le procédé comprenant les étapes consistant à :
acquérir (310) des informations caractéristiques incluant un statut de stérilisation chirurgicale et des informations sur les résultats de l'analyse du microbiome d'un individu cible ; et
déterminer (320) un aliment personnalisé pour l'individu cible sur la base des informations caractéristiques acquises et des informations sur les résultats de l'analyse du microbiome acquises,
dans lequel l'étape consistant à déterminer détermine, en utilisant un modèle de recommandation alimentaire entraîné, un aliment optimal personnalisé pour l'individu cible sur la base des informations caractéristiques et des informations sur les résultats de l'analyse du microbiome de l'individu cible, et
dans lequel le modèle de recommandation alimentaire entraîné a été généré au préalable par apprentissage automatique sur la base de données d'apprentissage incluant, pour chacun de divers individus, des caractéristiques comprenant un statut de stérilisation chirurgicale ; un résultat de l'analyse du microbiome ; et des aliments correspondant aux caractéristiques et au résultat de l'analyse du microbiome de cet individu.

9. Procédé selon la revendication 8, dans lequel les informations caractéristiques comprennent un type, une race, un poids, un âge, un sexe, un statut d'obésité, un statut de maladie et un résultat d'analyse sanguine.

10. Procédé selon la revendication 8, dans lequel l'étape consistant à déterminer (320) l'aliment personnalisé pour l'individu cible comprend :
la détermination des nutriments nécessaires à l'individu cible sur la base des informations caractéristiques acquises et des informations sur les résultats de l'analyse du microbiome acquises ; et
la détermination d'une composition alimentaire capable de fournir les nutriments déterminés.

11. Procédé selon la revendication 10, dans lequel la composition alimentaire comprend un type, une qualité et une quantité de composants de l'aliment.

12. Procédé selon la revendication 10, dans lequel l'étape consistant à déterminer (320) la composition alimentaire comprend :
la détermination d'un état de santé de l'individu cible sur la base des informations caractéristiques acquises et des informations sur les résultats de l'analyse du microbiome acquises ; et
lorsque l'état de santé déterminé est anormal, la détermination de types de bactéries nocives et de bactéries bénéfiques et d'un rapport entre ces bactéries chez l'individu cible en utilisant les informations sur les résultats de l'analyse du microbiome, la détermination de types et d'une quantité de bactéries bénéfiques nécessaires à l'individu cible, et la détermination de la composition alimentaire en tenant compte des types et de la quantité de bactéries bénéfiques nécessaires à l'individu cible.

13. Procédé selon la revendication 8, comprenant en outre l'étape consistant à :
fournir des informations sur l'aliment déterminé.

14. Procédé selon la revendication 13, dans lequel les informations sur les aliments comprennent un type d'aliment, des informations sur le produit et un mode d'alimentation.
